(19) Europäisches Patentamt
European Patent Office
Office européen des brevets

(11) EP 4 129 342 B1

(12) EUROPEAN PATENT SPECIFICATION

(45) Date of publication and mention
of the grant of the patent:
15.10.2025 Bulletin 2025/42

(21) Application number: 21780021.8

(22) Date of filing: 29.03.2021

(51) International Patent Classification (IPC):
A61K 47/26 (2006.01)      A61K 9/20 (2006.01)
A61K 47/32 (2006.01)      A61K 47/36 (2006.01)
A61K 47/38 (2006.01)

(52) Cooperative Patent Classification (CPC):
A61K 9/2018; A61K 9/2027; A61K 9/2054

(86) International application number:
PCT/JP2021/013162

(87) International publication number:
WO 2021/200767 (07.10.2021 Gazette 2021/40)

(54) **TABLET, MEDICINE, AND METHOD AND KIT FOR MANUFACTURING SAID TABLET AND MEDICINE**

TABLETTE, MEDIKAMENT SOWIE VERFAHREN UND KIT ZUR HERSTELLUNG DER BESAGTEN TABLETTE UND DES MEDIKAMENTS

COMPRIMÉ, MÉDICAMENT, ET PROCÉDÉ ET KIT POUR FABRIQUER LEDIT COMPRIMÉ ET LEDIT MÉDICAMENT

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 31.03.2020 JP 2020062818

(43) Date of publication of application:
08.02.2023 Bulletin 2023/06

(73) Proprietor: Eisai R&D Management Co., Ltd.
Tokyo 112-8088 (JP)

(72) Inventors:
• SANO, Shusuke
Kakamigahara-shi, Gifu 501-6195 (JP)
• ARASE, Shuntaro
Kakamigahara-shi, Gifu 501-6195 (JP)
• SHIKATA, Futoshi
Kakamigahara-shi, Gifu 501-6195 (JP)

(74) Representative: Hoffmann Eitle
Patent- und Rechtsanwälte PartmbB
Arabellastraße 30
81925 München (DE)

(56) References cited:
EP-A1- 2 433 620      WO-A1-2007/076874
JP-A- 2001 213 765      JP-A- 2001 213 765
JP-A- 2009 522 315      JP-A- H08 104 650
JP-A- S55 163 175      US-A1- 2006 134 202

## Description

## Technical Field

The present invention relates to a tablet and a medicine, methods for producing these, and to a kit, as set out in the appended set of claims.

## Background Art

A step for mixing active pharmaceutical ingredients with diluents, binders and the like and tableting the mixture is known as a conventional producing method of tablet. Such a method allows all of the intended active pharmaceutical ingredients to be included in the produced tablets.

As a new tablet producing method, Non-Patent Document 1 discloses a method in which a liquid loadable tablet (LLT) containing magnesium aluminometasilicate, crospovidone and magnesium stearate and a self-microemulsifying drug delivery system containing cyclosporin A (CyA-SMEDDS) are prepared, and the LLT is then immersed in an excess of the CyA-SMEDDS. In such a method, the originally prepared LLT does not contain the active pharmaceutical ingredient (cyclosporin), which is added to the LLT later.

Patent Document 1 discloses a blank tablet and a method of producing a blank tablet to which an active ingredient can be added. The blank tablet contains from about 0.1 to about 2 wt% of absorbent, from about 50 to about 95 wt% of diluent or binder, from about 0.5 to about 5 wt% of disintegrant, and from about 0.1 to about 2 wt% of lubricant. The diluent is a monosaccharide or a disaccharide, the binder is microcrystalline cellulose or compressed lactose and the disintegrant is sodium starch glycolate, sodium carboxymethyl cellulose or carboxymethyl cellulose calcium. Patent Document 2 discloses a disintegrating tablet product solely containing inert pharmaceutically acceptable excipients in compressed form and when the tablet is subjected to a pharmaceutically acceptable liquid formulation e.g. containing the active substance, the tablet will due to its porosity suck the liquid formulation into the tablet.

Patent Document 3 discloses a sublingual dosage forms that may be produced by impregnating (medicating) an inert tablet with at least one drug. The inert portion of the tablet comprises at least one excipient such as lactose, sucrose or a mixture of lactose and sucrose, at least one binder and a lubricant.

Patent Document 4 discloses a novel pharmaceutical composition of N-(3-methoxy-5-methylpyrazin-2-yl)-2-(4-[1,3,4-oxadiazol-2-yl]phenyl)pyridine-3-sulphonamide with mannitol and/or microcrystalline cellulose.

Citation List

Non-Patent Document

Non-Patent Document 1: AAPS PharmSciTech, Vol. 10, No. 4, pp. 1388-1395 (2009)

Patent Documents

Patent Document 1: JP 2001 213765 A

Patent Document 2: WO 2007/076874 A1

Patent Document 3: US 2006/134202 A1

Patent Document 4: EP 2 433 620 A1

## Summary

Technical Problem

A method for adding active pharmaceutical ingredients to tablets afterward elicits various advantages over conventional tablet producing methods, but because the silica of the porous inorganic particles used in the prior art has silanol groups on its surface, the silica may interact with the active pharmaceutical ingredient and other ingredients, and moreover the carrier itself is insoluble. Thus, because the active pharmaceutical ingredient adsorbed inside the carrier can only be released by natural dispersion in liquid, the release properties are poor in comparison with conventional tablets that release their ingredients when the tablet disintegrates or the ingredients dissolve. There is therefore demand for the development of a new tablet that would employ such a method without using porous inorganic particles of silica, which have various problems. Such a tablet may preferably have the properties of ordinary tablets, be permeable to active pharmaceutical ingredients, and have the property of retaining the permeated active pharmaceutical ingredients.

Thus, it is an object of the present invention to provide a tablet with excellent permeability and retention ability of active pharmaceutical ingredients, a medicine using this, and methods for producing the tablet and the medicine, as well as a kit.

Solution to Problem

The inventors and others found as a result of diligent research that a tablet comprising a monosaccharide and/or a disaccharide has excellent permeability and retention ability of active pharmaceutical ingredients. The invention is as set out in the appended set of claims.

Advantageous Effects of Invention

The present invention, as set out in the appended set of claims, provides a tablet with excellent permeability and retention ability of an active pharmaceutical ingredient.

**Brief Description of Drawings**

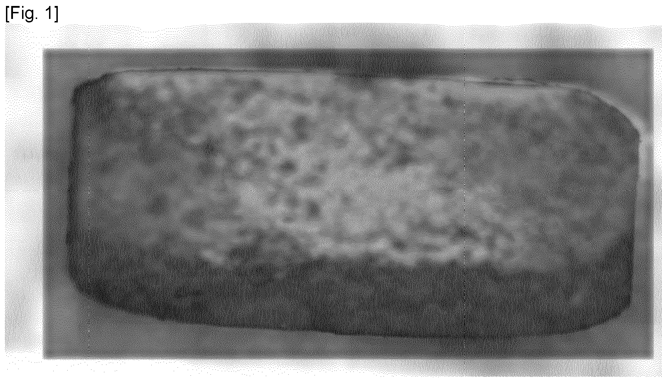

**Fig.** 1 is a Raman chemical image of a cross-section of an acetaminophen tablet.

**Description of Embodiments**

**<Tablet for impregnation>**

One embodiment of the present invention relates to a tablet for being impregnated with an active pharmaceutical ingredient (hereunder called a "tablet for impregnation"), comprising a monosaccharide and/or disaccharide and a water/insoluble polymer, wherein the monosaccharide and/or disaccharide comprises $\delta$-form D-mannitol. Using a monosaccharide and/or disaccharide makes it easier to impregnate the tablet for impregnation with an active pharmaceutical ingredient, and easier for the impregnated active pharmaceutical ingredient to be retained in the tablet for impregnation.

In this Description, a "tablet for impregnation" is a tablet that is to be impregnated later with an active pharmaceutical ingredient. The tablet for impregnation may comprise no active pharmaceutical ingredient, or may already comprise some of the active pharmaceutical ingredient to be impregnated later, or may comprise only some kinds of pharmaceutical ingredients when the medicine (final product) comprises two or more kinds of active pharmaceutical ingredients. When an active pharmaceutical ingredient is added to the pre-produced tablet for impregnation, it can then be used as a medicine. Advantages of this method of adding an active pharmaceutical ingredient to a tablet for impregnation include the ease of changing the content of the active pharmaceutical ingredient, the ability to reliably compound a tiny amount of a highly active pharmaceutical ingredient into each tablet for impregnation in the desired amount, the ability to easily compound multiple active pharmaceutical ingredients, the low risk the that active pharmaceutical ingredients that are unstable with respect to heat and the like will decompose during the producing process, the ability to avoid interactions between the active pharmaceutical ingredient and other ingredients, resistance to producing scale effects, and the ability to produce minimal required amounts of investigational drugs and drugs for rare diseases by preparing tablets for impregnation in advance.

The tablet for impregnation is used to produce a tablet (medicine) comprising an active pharmaceutical ingredient. Once the tablet for impregnation has been brought into contact with a drug solution comprising an active pharmaceutical ingredient, the solvent of the drug solution can be removed by drying to obtain a tablet (medicine) comprising the active pharmaceutical ingredient.

The tablet for impregnation can absorb a certain amount of a drug solution when brought into contact with the drug solution, and all or part the absorbed drug solution can be retained within the tablet for impregnation for a certain period of time.

Having been brought into contact with the drug solution comprising the active pharmaceutical ingredient, the tablet for impregnation can absorb a certain amount of the drug solution without apparently disintegrating (while retaining its external shape) and can retain the drug solution until the drying step is complete.

In order to incorporate a predetermined amount of the active pharmaceutical ingredient into the tablet for impregnation, the concentration and amount of the drug solution brought into contact with the tablet for impregnation may be determined so that the drug solution comprising a predetermined amount of the active pharmaceutical ingredient does not leak out of the tablet for impregnation. Alternatively, the tablet for impregnation may be immersed in a drug solution of a specific concentration and the impregnation time may be set so as to adjust the amount of the drug solution absorbed by the tablet for impregnation (amount of drug in medicine after drying).

A rapid disintegrating tablet having the mechanical strength (hardness, friability) of a conventional pharmaceutical product and the ability to disintegrate rapidly in contact with water can be produced using the tablet for impregnation. An orally disintegrating tablet comprising an active pharmaceutical ingredient can also be produced using the tablet for impregnation.

Monosaccharides and disaccharides can improve the permeability, retention ability, strength and disintegration properties of the tablet for impregnation. To ensure excellent permeability, retention ability, strength and disintegration properties, the content of the monosaccharide and/or disaccharide is preferably at least 60% by mass, or more preferably at least 70% by mass, or still more preferably at least 80% by mass, or especially at least 90% by mass based on the mass of the tablet for impregnation. The maximum content of the monosaccharide and/or disaccharide is not especially limited, but may be 100% by mass, or 98% by mass, or 96% by mass, or 94% by mass for example.

The concept of monosaccharides and disaccharides includes their respective sugar alcohols, and for example lactose, sucrose, trehalose, maltose, mannitol, erythritol, xylitol, sorbitol and maltitol are preferred, and $\delta$-form D-mannitol is more preferred.

The tablet for impregnation also comprises a water-insoluble polymer. In this Description, "water-insoluble" means "almost insoluble" as indicated by the general rules of the 17th Revised Japanese Pharmacopoeia (that is, the "amount of solvent required to dissolve 1 g or 1 ml of solute" is "at least 10,000 ml"). By including a water-insoluble polymer, it is possible to improve permeability and disintegration properties while maintaining the retention ability and strength of the tablet for impregnation. Examples of water-insoluble polymers include croscarmellose sodium, carmellose, crospovidone, microcrystalline cellulose, low-substituted hydroxypropyl cellulose, carmellose calcium, partially pregelatinized starch, sodium starch glycolate and the like. These are commonly used as disintegrants. One kind of water-insoluble polymer or a combination of multiple kinds may be used.

So that the tablet for impregnation can maintain its shape when impregnated with an active pharmaceutical ingredient that has been dissolved or dispersed in an organic solvent such as ethanol for example, the water-insoluble polymer is preferably insoluble or hardly soluble in this organic solvent. In this Description, "hardly soluble" means "extremely hard to dissolve" (that is, the amount of solvent required to dissolve 1 g or 1 ml of solute is from 1,000 ml to less than 10,000 ml), "hard to dissolve" (that is, the amount of solvent required to dissolve 1 g or 1 ml of solute is from 100 ml to less than 1,000 ml), or "somewhat hard to dissolve" (that is, the amount of solvent required to dissolve 1 g or 1 ml of solute is from 30 ml to less than 100 ml) as indicated by the general rules of the 17th Revised Japanese Pharmacopoeia.

In addition to ethanol, other examples of organic solvents here include isopropyl alcohol, 1-propanol, methanol and DMF.

**To** ensure excellent permeability, retention ability, strength and disintegration properties, the content of the water-insoluble polymer is preferably from 0.1 to 30% by mass, or more preferably from 0.5 to 20% by mass, or still more preferably from 1 to 15% by mass based on the mass of the tablet for impregnation.

The tablet for impregnation preferably also comprises a binder, and more preferably comprises a binder together with a water-insoluble polymer. The binder can improve the permeability and disintegration properties while maintaining the retention ability and strength of the tablet for impregnation. Examples of the binder include polyvinyl alcohol (which may be partially saponified), polyvinyl pyrrolidone (povidone), hypromellose, pullulan, xanthan gum, guar gum, gelatin, carrageenan and agar. One kind of binder or a combination of multiple kinds may be used. So that the tablet for impregnation can maintain its shape when impregnated with an active pharmaceutical ingredient that has been dissolved or dispersed in an organic solvent such as ethanol, the binder is preferably one that is insoluble or hardly soluble in this organic solvent. However, polyvinyl pyrrolidone and hypromellose can be used favorably even though they are soluble in ethanol.

In addition to ethanol, other examples of organic solvents here include isopropyl alcohol, 1-propanol, methanol and DMF.

To ensure excellent permeability, retention ability, strength and disintegration properties, the content of the binder is preferably from 0.1 to 10% by mass, or more preferably from 0.3 to 8% by mass, or still more preferably from 0.6 to 6% by mass based on the mass of the tablet for impregnation.

The tablet for impregnation may also comprise optional ingredients to the extent that the effects are not adversely affected. Examples of optional ingredients include excipients, lubricants, fluidizers, colorants and the like.

Examples of excipients include corn starch, calcium carbonate, anhydrous calcium hydrogen phosphate and the like.

Examples of lubricants include magnesium stearate, calcium stearate, talc, hydrogenated vegetable oil, sucrose fatty acid esters, polyethylene glycol, sodium lauryl sulfate and the like.

Examples of fluidizers include light silicic anhydride, hydrous silicon dioxide, titanium oxide, synthetic aluminum silicate, magnesium aluminometasilicate and the like.

Examples of colorants include tar dyes, natural dyes and the like.

The properties of the tablet for impregnation may be changed by adjusting its theoretical porosity. For example, to improve the permeability, retention ability, strength and disintegration properties of the tablet for impregnation, the theoretical porosity is preferably from 20% to 50%, or more preferably from 25% to 45%, or still more preferably from 30% to 40%.

The theoretical porosity can be increased by using a high-density composition and compressing and molding at low pressure to increase the tablet volume, but at the same time the molded product also needs to have the properties of hardness and wear resistance required of a tablet.

The theoretical porosity can be calculated using the following formula (1).

[Math. 1]

$$\text{Theoretical porosity (\%)} = (1 - \frac{solid\ volume\ (cm3)}{tablet\ volume\ (cm3)}) \times 100 \qquad \text{Formula (1)}$$

The solid volume can be calculated based on the following formula (2).

[Math. 2]

Solid volume $(cm^3)$ = total of (formulated mass of each ingredient (g/tablet) ÷ true density of each ingredient $(g/cm^3)$) 

Formula (2)

The tablet volume can be measured using a known volume measuring instrument such as a 3D scanner (Keyence Corp. VL Series). The tablet volume can also be easily calculated based on the following formula (3).

[Math, 3]

$$\text{Tablet volume } (cm^3) = (\text{tablet diameter (cm)} \div 2)^2 \times \pi \times \text{tablet thickness (cm)}$$

Formula (3)

Formula (3) above is an easy method of calculation assuming that the tablet for impregnation has a cylindrical shape. When the tablet for impregnation has a cylindrical shape with angular edges and the volume of the angular edges needs to be calculated, this can be calculated and corrected using Pappus-Guldin's theorem. When the tablet for impregnation is not cylindrical, the volume can be easily calculated using a different formula.

The shape of the tablet for impregnation is not particularly limited, but preferably the tablet has a surface indentation. With this indentation, it is possible to avoid the problem of overflow from the tablet for impregnation when a solution or dispersion comprising an active pharmaceutical ingredient is dripped onto the tablet.

<Producing method for tablet for impregnation>

One embodiment of the present invention relates to a method for producing a tablet for impregnation, which includes a mixing step and a tableting step.

The mixing step is a step of mixing a monosaccharide and/or disaccharide and a water-insoluble polymer with a solvent to obtain a mixture, wherein the monosaccharide and/or disaccharide comprises δ-form D-mannitol. Preferably a binder is also mixed in the mixing step. The details of the monosaccharide and disaccharide, water-insoluble polymer and binder are as explained above under "Tablet for impregnation". The mixture is preferably granulated in the mixing step.

Examples of the solvent used in the mixing step include water, ethanol, isopropyl alcohol, 1-propanol, methanol and DMF. Although this is not a limitation, it is desirable to include at least water as a solvent in order to improve the retention ability and strength, and more desirable to use a mixed solvent of water and ethanol. When using a mixed solvent of water and ethanol, the mass ratio of the water and ethanol is preferably from 1:0.1 to 1:10, or more preferably from 1:0.3 to 1:5, or still more preferably from 1:0.6 to 1:3.

The amount of the solvent used is preferably from 1 to 40% by mass, or more preferably from 10 to 30% by mass, or still more preferably from 15 to 25% by mass based on the total mass of the constituent ingredients of the tablet for impregnation (excluding the solvent).

Optional ingredients may also be used in the mixing step as long as the effects of the tablet for impregnation are not adversely affected. The details of the optional ingredients are as explained above under "Tablet for impregnation".

Mixing methods include for example wet granulation, dry granulation and the like. Although this is not a particular limitation, wet granulation is preferred in order to give the tablet for impregnation both high porosity and the properties necessary for a tablet.

Commonly used equipment and techniques such as high shear granulator (Kawata SMV-20A) or fluidized bed granulation can be used as the wet granulation methods.

**The** tableting step is a step of tableting the mixture obtained in the mixing step. The tableting step is preferably performed on the mixture in a wet state.

Commonly used equipment and techniques using a single-punch tableting device (Autograph, Shimadzu Corp.), rotary tableting device, rapid disintegrating tablet molding machine (EMT-form molding machine, Sankyo Seisakusho) or the like may be used for the tableting method.

**The** producing method of this embodiment may also include a drying step after the tableting step. The drying step is a step to remove residual solvent from the produced tablet for impregnation.

Examples of the drying method include natural drying, drying methods by air flow or hot air using a shelf drier, ventilation drier or transport drier (EDT-form drier, Sankyo Seisakusho), and freeze drying, vacuum drying, microwave drying and the like.

<Medicine>

One embodiment of the invention relates to a medicine comprising an active pharmaceutical ingredient impregnated in (preferably dripped onto) a tablet for impregnation, as described herein. In the case of a medicine obtained by subsequently adding an active pharmaceutical ingredient to a tablet for impregnation, the active pharmaceutical ingredient may be non-uniformly present in the tablet. Thus, the medicine can be described as a medicine comprising a tablet for impregnation, as described herein, and an active pharmaceutical ingredient present in the tablet for impregnation, in which the active pharmaceutical ingredient is present non-uniformly in the tablet for impregnation.

**The** non-uniform presence of the active pharmaceutical ingredient is due to the active pharmaceutical ingredient having been added after the fact to the tablet for impregnation, as disclosed herein. In the case of a medicine obtained by dripping an active pharmaceutical ingredient for example, there is likely to be more of the active pharmaceutical ingredient on the surface part (dripping part) of the tablet for impregnation where the active pharmaceutical ingredient has been dripped. This means that the active pharmaceutical ingredient in the dripping part is more abundant than the active pharmaceutical ingredient in the part corresponding to the dripping part in a tablet in which the same content of the active pharmaceutical ingredient is distributed uniformly, while the amount of the active pharmaceutical ingredient is smaller in the areas apart from the dripping part. Therefore, confirming the distribution of the active pharmaceutical ingredient is one method of verifying the method of producing the medicine.

"Non-uniformly present" may mean for example that in a tablet for impregnation that has been impregnated with an active pharmaceutical ingredient by immersion, the amount of the active pharmaceutical ingredient present in the surface part of the tablet for impregnation (hereunder called the "surface component") differs from the amount of the active pharmaceutical ingredient present in the central part of the tablet for impregnation (hereunder called the "central component"), so that the pharmaceutically active ingredient is distributed preferentially near the surface part or the like.

Alternatively, it may mean that in a tablet for impregnation that has been impregnated with an active pharmaceutical ingredient by dripping, the amount of the active pharmaceutical ingredient present in the dripping part (hereunder called the "dripping part component") is greater than the amount of the active pharmaceutical ingredient present on the surface opposite the dripping part (hereunder called the "opposite part component"), so that the pharmaceutically active ingredient is distributed preferentially in the dripping part.

One method for confirming the uneven distribution of the surface component and central component or the dripping part component and opposite part component is by spectrum analysis using Raman spectroscopy. Using a cross-sectional image (cross-section cut perpendicular to the surface on which the drug solution has been dripped) of a medicine comprising a tablet for impregnation impregnated with an active pharmaceutical ingredient, the preferential distribution of the active pharmaceutical ingredient near the surface part can be confirmed by displaying superimposed image showing the distribution of the active pharmaceutical ingredient detected by spectrum analysis using Raman spectroscopy.

**The** type of active pharmaceutical ingredient is not particularly limited, but examples include active pharmaceutical ingredients that are prescribed in finely graded doses depending on the patient's body weight, and active pharmaceutical ingredients for administration to elderly patients who are taking many different medications for many different conditions. Advantages include the ability to adjust the dosage appropriately on the spot according to the patient's body weight and the ability to administer multiple medications in a single tablet, thereby reducing the bother of taking many different tablets and contributing to drug compliance. Consequently, there may be one kind or multiple kinds of active pharmaceutical ingredients. The active pharmaceutical ingredient may be an active ingredient used in a quasi-drug, and in this case the medicine of this embodiment includes quasi-drugs. The active pharmaceutical ingredient may also be a nutritional ingredient, or a ingredient associated with a functional food, and in this case the medicine of this embodiment includes functional foods containing such ingredients. The content of the active pharmaceutical ingredient is adjusted appropriately according to the dosage.

To improve the rapid disintegrating property of the medicine, the theoretical porosity of the medicine is preferably from 20% to 50%, or more preferably from 25% to 45%, or still more preferably from 30% to 40%. Because the theoretical porosity of the medicine depends on the theoretical porosity of the tablet for impregnation, the theoretical porosity of the medicine can be adjusted by adjusting the theoretical porosity of the tablet for impregnation. The theoretical porosity of the medicine can be determined by a method similar to that used to determine the theoretical porosity of the tablet for impregnation.

The medicine may also have a coating layer on its surface. Examples of types of coating layers include sugar coatings, film coatings and the like.

<Method for producing medicine>

One embodiment of the invention relates to a medicine producing method including an impregnation step.

The impregnation step is a step of impregnating a tablet for impregnation, as described herein, with an active pharmaceutical ingredient. In this Description, "impregnating" means causing the tablet for impregnation to comprise the active pharmaceutical ingredient. The method of impregnation is not particular limited but may for example be a method of dripping a solution or dispersion of the active pharmaceutical ingredient onto the tablet for impregnation (hereunder called a "dripping method") or a method of immersing the tablet for impregnation in such a solution or dispersion (hereunder called an "immersion method"). A dripping method is preferred from the standpoint of reliably impregnating with the specified amount of the active pharmaceutical ingredient.

The solvent of the solution or dispersion comprising the active pharmaceutical ingredient is not particularly limited as long as it is pharmacologically acceptable (that is, a solvent with low toxicity). Examples of pharmacologically acceptable solvents include ethanol, isopropanol, 1-propanol, methanol and DMF. Ethanol is desirable for ensuring a high degree of safety.

The tablet for impregnation used in the impregnation step is as described herein and has a specific permeability and retention ability. The concentration and amount of the solution or dispersion comprising the active pharmaceutical ingredient may be adjusted appropriately according to this permeability and retention ability so that the desired active pharmaceutical ingredient can be compounded in the medicine. In the case of a dripping method, producing control is easier if the concentration and amount are adjusted so that the dripped solution or dispersion can rapidly permeate without seeping from the tablet for impregnation and be retained for a specific period of time (such as the time between dripping and the beginning of the drying step) in the tablet for impregnation.

In this Description, "permeability" means the ease with which the solution or dispersion comprising the active pharmaceutical ingredient permeates the tablet for impregnation. From the standpoint of producing efficiency, it is desirable that the solution or dispersion comprising the active pharmaceutical ingredient be able to rapidly permeate inside the tablet for impregnation. The permeation time described in the examples below may be used as a benchmark of permeability. While a shorter permeation time is advantageous, it is sufficient that the solution or dispersion have permeated the tablet for impregnation during the transfer to the drying step, and a benchmark of within 120 seconds may be adopted. On the other hand, a short permeation time of about 10 seconds may make it difficult to control the producing process conditions if retention ability is lacking and there is rapid seepage from the tablet.

"Retention ability" in this Description means that the solution or dispersion of the active pharmaceutical ingredient that has been impregnated in the tablet for impregnation can be retained without seeping from the tablet. As shown in the examples below, when a certain quantity of a dispersion or solution has been dripped onto a tablet for impregnation the retention ability of the tablet for impregnation can be evaluated qualitatively by confirming whether or not the solution or dispersion has been retained and the degree of retention. A tablet for impregnation that easily retains a solution or dispersion in quantity is advantageous for expanding the range of producing conditions for the medicine of this embodiment. The concentration or amount of the solution or dispersion may be adjusted to suit the confirmed retention ability when setting the producing conditions, a viscosity adjuster may also be used to adjust the viscosity of the solution or dispersion, and other process conditions may also be adjusted. The retention ability of the tablet for impregnation can also be evaluated quantitively by changing the amount of the solution or dispersion dripped on the tablet for impregnation and determining the amount of liquid retained when seepage begins (maximum amount that can be retained). Although a tablet with a greater retention capacity is advantageous for producing the medicine, a certain degree of retention ability is sufficient because the compounded amount of the active pharmaceutical ingredient can also be controlled by controlling the conditions of the solution or suspension and the like.

The tablet for impregnation, as described herein, may have a hardness sufficient to withstand the impregnation step and subsequent steps, or a hardness sufficient to allow the medicine produced by these steps to withstand impacts during distribution.

The producing method of this embodiment may also include a drying step following the impregnation step. The drying step is a step of removing residual solvent from the produced medicine.

Methods of drying include natural drying, drying methods by air flow or hot air using a shelf drier, ventilation drier or transport drier (EDT-type drier, Sankyo Seisakusho), and freeze drying, vacuum drying and the like.

**The** producing method of this embodiment may also include a coating step following the drying step. The coating step is a step of forming a coating layer on the surface of the medicine. The details of the coating layer are as explained above under "Medicine".

Examples of coating methods include pan coating in which the tablet is placed in a coating pan and rolled to uniformly coat the entire tablet, and spray coating in which a coating agent is partially applied with a spray nozzle to a standing tablet. The coating layer may cover the entire tablet or only part of the tablet.

Advantages of the producing method of this embodiment include the ease of changing the content of the active pharmaceutical ingredient, the ability to reliably compound a tiny amount of a highly active pharmaceutical ingredient into the tablet for impregnation in the desired amount, the ability to avoid interactions between the active pharmaceutical ingredient and other ingredients, the low risk the that active pharmaceutical ingredients that are unstable with respect to heat and the like will break down during the producing process, the ability to avoid interactions between the active pharmaceutical ingredient and other ingredients, resistance to producing scale effects, and the ability to produce minimal required amounts of experimental drugs and drugs for rare diseases by preparing tablets for impregnation in advance.

<Kit>

One embodiment of the invention relates to a kit comprising a tablet for impregnation, as described herein, and an active pharmaceutical ingredient. The kit may also contain a solvent for dissolving or dispersing the active pharmaceutical ingredient. The kit may also contain a manual explaining the method of use. Using the kit, it is possible to produce a medicine in the location where it will actually be used.

[Examples]

**The** present invention is explained in more detail below using examples and comparative examples, but these do not limit the technical scope of the invention.

<Evaluating tablet for impregnation>

(Permeability)

The tablets for impregnation produced in the examples and comparative examples below were evaluated for ethanol permeability (dripping ethanol permeation time). The permeation time was the time taken from dripping 50 $\mu$l of ethanol onto the tablet surface (top of the tablet) until the ethanol drops completely disappeared from the tablet surface.

(Retention ability)

The ethanol retention ability of the tablets for impregnation produced in the examples and comparative examples below was measured. To evaluate retention ability, 50 $\mu$l of ethanol was dripped onto the tablet surface (top of the tablet), and once this had completely permeated, seepage of ethanol from the tablet surface (bottom of the tablet) was observed and retention was evaluated on a scale of A to C, in which A is the best and C is the worst.

(Theoretical porosity)

The theoretical porosities of the tablets for impregnation produced in the examples and comparative examples below were evaluated based on the above formulae (1) to (3).

(Hardness)

The hardness (N) of the tablets for impregnation produced in the examples and comparative examples below was measured using a hardness tester.

(Disintegration time)

The disintegration times of the tablets for impregnation produced in the examples and comparative examples below were measured. Disintegration time was evaluated using purified water according to the general disintegration test methods of the Japanese Pharmacopoeia.

<Producing and testing tablets for impregnation>

(Reference Example 1) to (Reference Example 4)

0.6 g of purified water (RO water) was mixed with 0.6 g of dehydrated ethanol (manufactured by Kanto Chemical), placed in a mortar, mixed with 6.0 g of δ-form D-mannitol (Merck, Parteck Delta M), and granulated for 3 minutes with the mortar to obtain a granulated product. The granulated product was crushed with a sieve (JP 16, 1,000 μm mesh) to obtain wet granules. The wet granules were tableted with a single-punch tableting device (Autograph, manufactured by Shimadzu) to a tablet diameter of 8 mm and a tablet mass of 150 mg, and dried at 60°C to obtain a tablet for impregnation.
Four kinds of tablets for impregnation were obtained by adjusting the tableting pressure during tableting to prepare tablets with different theoretical porosities. These tablets for impregnation were Reference Examples 1 to 4 in order from the lowest theoretical porosity.

(Test Reference Examples 1 to 4)

The Reference Examples 1 to 4 were evaluated for permeability, retention ability, hardness and disintegration time. The theoretical porosity was also calculated. The results are shown in Table 1.
The results show that all of Reference Examples 1 to 4 had good permeability and retention ability (A). They also had the properties of hardness and disintegration time required of a tablet. In terms of theoretical porosity, it appears that the permeability is better the higher the theoretical porosity.

(Example 1)

5.7 g of δ-form D-mannitol (Merck, Parteck Delta M) and 0.3 g of low-substituted hydroxypropyl cellulose (Shin-Etsu Chemical Co., Ltd., LH-31) were placed in a mortar and mixed for 3 minutes with the mortar. A mixed solvent of 0.6 g of purified water and 0.6 g of dehydrated ethanol (Kanto Chemical) was added in its entirety to the resulting mixture, which was then granulated for 3 minutes with the mortar to obtain a granulated product. The granulated product was crushed with a sieve (JP 16, 1,000 μm mesh) to obtain a wet granules. The wet granules were tableted with a single-punch tableting device (Autograph, Shimadzu) to a tablet diameter of 8 mm and a tablet mass of 150 mg, and dried at 60°C to obtain a tablet for impregnation.

(Test Example 1)

The Example 1 was evaluated for permeability, retention ability, hardness and disintegration time. The theoretical porosity was also calculated. The results are shown in Table 1.
The results show that with the Example 1 also comprising low-substituted hydroxypropyl cellulose (LH-31), good retention ability (A) was maintained, and permeability was improved in comparison with Reference Examples 1 to 4. The disintegration time was also shorter, and good hardness results were obtained.

(Example 2)

6 g of (partially saponified) polyvinyl alcohol (Nippon Synthetic Chemical Industry Co., Ltd. Gohsenol™ EG-05) was dissolved in 30 g of purified water to obtain a 16.7% polyvinyl alcohol solution. 0.3 g of purified water and 0.6 g of dehydrated ethanol (Kanto Chemical) were mixed with 0.36 g of the 16.7% polyvinyl alcohol solution to obtain a binding solution. 5.64 g of δ-form D-mannitol (Merck, Parteck Delta M) and 0.3 g of low-substituted hydroxypropyl cellulose (Shin-Etsu Chemical Co., Ltd., LH-31) were placed in a mortar and mixed for 3 minutes with the mortar. All of the binding solution was added to the resulting mixture, which was then granulated for 3 minutes with the mortar to obtain a granulated product. The granulated product was crushed with a sieve (JP 16, 1,000 μm mesh) to obtain wet granules. The wet granules were tableted with a single-punch tableting device (Autograph, Shimadzu) to a tablet diameter of 8 mm and a tablet mass of 150 mg, and dried at 60°C to obtain a tablet for impregnation.

(Example 3)

0.6 g of povidone (manufactured by ISP, Plasdone™ K-29/32) was dissolved in 1.2 g of a mixed solution of purified water and dehydrated ethanol (50% mixed solution) to obtain a binding solution. A tablet for impregnation was then produced under the same conditions as Example 2 using this binding solution following the compositional table of Table 1.

(Test Examples 2 and 3)

The Examples 2 and 3 were evaluated for permeability, retention ability, hardness and disintegration time. The theoretical porosity was also calculated. The results are shown in Table 1.

The results show that with the Examples 2 and 3 also comprising binders, good permeability and retention ability (A) were maintained, and better results were obtained in Comparison with Example 1 in terms of shorter disintegration times. Good hardness results were also obtained.

(Example 4)

Low-substituted hydroxypropyl cellulose (LH-11) was substituted for the low-substituted hydroxypropyl cellulose (LH-31) of Example 2, and a tablet for impregnation was produced under the same conditions as Example 2 following the compositional table of Table 1.

(Example 5)

Low-substituted hydroxypropyl cellulose (LH-21) was substituted for the low-substituted hydroxypropyl cellulose (LH-31) of Example 2, and a tablet for impregnation was produced under the same conditions as Example 2 following the compositional table of Table 1.

(Example 6)

Croscarmellose sodium (FMC International Inc., Ac-Di-Sol) was substituted for the low-substituted hydroxypropyl cellulose of Example 2, and a tablet for impregnation was produced under the same conditions as Example 2 following the compositional table of Table 1.

(Example 7)

Carmellose (Gotoku Chemical NS-300) was substituted for the low-substituted hydroxypropyl cellulose of Example 2, and a tablet for impregnation was produced under the same conditions as Example 2 following the compositional table of Table 1.

(Example 8)

Crospovidone (manufactured by ISP, Polyplasdone XL-10) was substituted for the low-substituted hydroxypropyl cellulose of Example 2, and a tablet for impregnation was produced under the same conditions as Example 2 following the compositional table of Table 1.

(Example 9)

Microcrystalline cellulose (manufactured by Asahi Kasei, Japanese Pharmacopoeia Ceolus™ PH-101) was substituted for the low-substituted hydroxypropyl cellulose of Example 2, and a tablet for impregnation was produced under the same conditions as Example 2 following the compositional table of Table 1.

(Test Examples 4 to 9)

The Examples 4 to 9 were evaluated for permeability, retention ability, hardness and disintegration time. The theoretical porosity was also calculated. The results are shown in Table 1.

The results show that good permeability and retention ability (A) were obtained with the examples 4 to 9 comprising water-insoluble polymers other than the low-substituted hydroxypropyl cellulose (LH-31) of Example 2. Good results were also obtained in terms of disintegration time and hardness.

(Example 10)

0.6 g of hypromellose 2910 (Shin-Etsu Chemical, TC-5E Japanese Pharmacopoeia Hypromellose) was dissolved in 1.2 g of a mixed solution of sterile purified water and dehydrated ethanol (50% mixed solution) to obtain a binding solution. A tablet for impregnation was obtained under the same conditions as Example 2 using this binding solution following the compositional table of Table 1.

(Example 11)

0.6 g of pullulan (Hayashibara Co., Ltd. Japanese Pharmacopoeia Pullulan) was dissolved in 1.2 g of a mixed solution of sterile purified water and dehydrated ethanol (50% mixed solution) to obtain a binding solution. A tablet for impregnation was obtained under the same conditions as Example 2 using this binding solution following the compositional table of Table 1.

(Example 12)

0.6 g of xanthan gum (Danisco, Grindsted™ Xanthan J) was dissolved in 1.2 g of a mixed solution of sterile purified water and dehydrated ethanol (50% mixed solution) to obtain a binding solution. A tablet for impregnation was obtained under the same conditions as Example 2 using this binding solution following the compositional table of Table 1.

(Test Examples 10 to 12)

The Examples 10 to 12 were evaluated for permeability, retention ability, hardness and disintegration time. The theoretical porosity was also calculated. The results are shown in Table 1.
The results show that good permeability and retention ability (A) were also obtained with the Examples 10 to 12 comprising binders different from the polyvinyl alcohol of Example 2. Good results were also obtained in terms of disintegration time and hardness.

(Example 13)

The amount of δ-form D-mannitol was changed to 5.34 g and the amount of low-substituted hydroxypropyl cellulose to 0.6 g in Example 2, and a tablet for impregnation was produced under the same conditions as Example 2 following the compositional table of Table 1.

(Reference Example 5)

The amount of δ-form D-mannitol was changed to 5.94 g and the low-substituted hydroxypropyl cellulose was omitted in Example 2, and a tablet for impregnation was produced under the same conditions as Example 2 following the compositional table of Table 1.

(Test Example 13 and Reference Example 5)

The Example 13 and Reference Example 5 were evaluated for permeability, retention ability, hardness and disintegration time. The theoretical porosity was also calculated. The results are shown in Table 1.
Also taking Example 2 into consideration, the results show that when a binder was included, good permeability and retention ability (A) were obtained when the content of the water-insoluble polymer was 5% or 10% of the total amount of the tablet for impregnation, and good results were also obtained in terms of disintegration time and hardness.
Good results in terms of permeability, disintegration time and hardness were also obtained in Example 20 comprising no water-insoluble polymer. However, a small amount of seepage was observed (retention ability (B)).

(Example 14)

A mixture of 0.72 g of 16.7% polyvinyl alcohol solution and 0.6 g of dehydrated ethanol was used as the binding solution. The amount of δ-form D-mannitol was also changed to 5.58 g in the Example 2, and a tablet for impregnation was produced under the same conditions as Example 2 following the compositional table of Table 1.

(Test Example 14)

The Example 14 was evaluated for permeability, retention ability, hardness and disintegration time. The theoretical porosity was also calculated. The results are shown in Table 1.
Also taking Examples 1 and 2 into consideration, the results show that when a water-insoluble polymer was included, good permeability and retention ability (A) were obtained when the content of the binder was 0%, 1% or 2% of the total amount of the tablet for impregnation, and good results were also obtained in terms of disintegration time and hardness.

(Example 15)

A mixture of 0.3 g of 16.7% polyvinyl alcohol, 0.25 g of purified water and 0.5 g of dehydrated ethanol was used as the binding solution. The amount of δ-form D-mannitol was also changed to 4.7 g and the amount of low-substituted hydroxypropyl cellulose to 0.25 g in the Example 2, and a tablet for impregnation was produced under the same conditions as Example 2 following the compositional table of Table 1.

(Example 16)

A mixture of 0.3 g of 16.7% polyvinyl alcohol and 0.75 g of purified water was used as the binding solution. The amount of δ-form D-mannitol was also changed to 4.7 g and the amount of low-substituted hydroxypropyl cellulose to 0.25 g in the Example 2, and a tablet for impregnation was produced under the same conditions as Example 2 following the compositional table of Table 1.

(Comparative Example 1)

4.7 g of δ-form D-mannitol (Merck, Parteck Delta M), 0.25 g of low-substituted hydroxypropyl cellulose (Shin-Etsu Chemical LH-31) and 0.05 g of (partially saponified) polyvinyl alcohol were placed in a mortar and mixed for 3 minutes with the mortar. 1.0 g of dehydrated ethanol was added to the resulting mixture, which was then granulated for 3 minutes with the mortar to obtain a granulated product. The granulated product was wet crushed with a sieve (JP 16, 1,000 μm mesh) to obtain a wet granulated product. Molding and drying were performed under the same conditions as Example 6 to produce a tablet.

(Test Examples 15 and 16 and Comparative Test Example 1)

The Examples 15 and 16 and the Comparative Example 1 were evaluated for permeability, retention ability and hardness. The theoretical porosity was also calculated. The results are shown in Table 1.
The results show that good permeability, retention ability (A) and hardness were obtained in Example 15 using a mixed solvent of sterile purified water and dehydrated ethanol.
Good results were also obtained in terms of permeability and hardness in the Example 16 using only sterile purified water as the solvent. However, a small amount of seepage was observed (retention ability (B)).
In the Comparative Example 1 using only dehydrated ethanol as the solvent, however, the quality of hardness required a tablet was very poor, and the tablet was fragile and unsuitable as a tablet for impregnation.

(Reference Example 6)

Sorbitol (Roquette Japan, Neosorb™ P650) was substituted for the δ-form D-mannitol of Example 2, and a tablet was produced under the same conditions as Example 2 following the compositional table of Table 1.

(Reference Example 7)

Lactose monohydrate (DMV, Pharmatose™ 200M) was substituted for the δ-form D-mannitol of Example 2, and a tablet was produced under the same conditions as Example 2 following the compositional table of Table 1.

(Reference Example 8)

β-form D-mannitol (Merck, Parteck M100) was substituted for the δ-form D-mannitol (Merck, Parteck Delta M) of Example 2, and a tablet was produced under the same conditions as Example 2 following the compositional table of Table 1.

(Test Reference Examples 6 to 8)

The Reference Examples 6 to 8 were evaluated for permeability, retention ability, hardness and disintegration time. The theoretical porosity was also calculated. The results are shown in Table 1.
The results show that good permeability, hardness and disintegration time were obtained in all of Reference Examples 6 to 8, in which sorbitol, lactose hydrate and β-form D-mannitol were used instead of δ-form D-mannitol. In terms of retention ability, a slight amount of seepage was observed in the Reference Examples 6, 7 and 8 (Retention ability (B)).

(Comparative Example 2)

3 g of (partially saponified) polyvinyl alcohol (Nippon Synthetic Chemical Industry Co., Ltd. Gohsenol™ EG-05) was dissolved in 30 g of purified water to obtain a 9.1% polyvinyl alcohol solution. 0.6 g of dehydrated ethanol (Kanto Chemical) was mixed with 0.66 g of the 9.1% polyvinyl alcohol solution to obtain a binding solution. 5.94 g of the polysaccharide microcrystalline cellulose (Asahi Kasei, Ceolus™ PH-101) was placed in a mortar, the above binding solution was added in its entirety, and this was granulated for 3 minutes with the mortar to obtain a granulated product. The granulated product was wet crushed with a sieve (JP 16, 1,000 μm mesh) to obtain a wet granulated product. The wet granulated product was tableted with a single-punch tableting device (Autograph, Shimadzu) to a tablet diameter of 8 mm and a tablet mass of 150 mg, and dried at 60°C to obtain a tablet for impregnation.

(Comparative Test Example 2)

The Comparative Example 2 was evaluated for permeability, retention ability and hardness. The theoretical porosity was also calculated. The results are shown in Table 1.
The results show that in Comparative Example 2 comprising the polysaccharide microcrystalline cellulose but no monosaccharide or disaccharide, most of the dripped ethanol seeped out of the tablet (Retention ability (C)), which could not be used as a tablet for impregnation.

<Producing and testing medicine>

(Example 17)

32.5 g of (partially saponified) polyvinyl alcohol was added to 650 g of heated sterile purified water (Yoshida Pharmaceutical) and dissolved by stirring with an air motor to obtain an aqueous polyvinyl alcohol solution. 25 g of dehydrated ethanol was added to 105 g of the aqueous polyvinyl alcohol solution and stirred to obtain 130 g of a binding solution. This was repeated 4 times to obtain a total of 520 g of binding solution. 2350 g of δ-form D-mannitol and 125 g of low-substituted hydroxypropyl cellulose were placed in a high shear granulator (Kawada Seisakusho, Supermix SMV-20) and mixed for 3 minutes at an impeller speed of 800 rpm. Mixing was continued as 130 g of the binding solution was added and granulated for 1 minutes. This was repeated 4 times to obtain a granulated product. The granulated product was crushed with a milling machine (Fukae Powtec TC-150) at an impeller speed of 800 rpm with a screen size of 4.0 mm φ to obtain wet granules. The tableting granules were molded at a molding pressure of 50-350N with a wet tableting machine (Sankyo Seisakusho EMT/ETD-18) to a diameter of 8.0 mm and a dried mass of 160 mg, and dried at 60°C to obtain tablets for impregnation.
100 mg of acetaminophen was dissolved in 800 mg of ethanol to obtain an acetaminophen solution. 42 mg of the resulting acetaminophen solution was dripped onto tablets for impregnation so that the solution penetrated the tablets. The tablets were then dried in a 50°C thermostatic chamber to obtain 5 mg tablets comprising acetaminophen.

(Example 18)

100 mg of cimetidine was dissolved in 600 mg of methanol to obtain a cimetidine solution. 35 mg of the cimetidine solution per tablet was dripped onto the tablets produced in Example 17 so that the solution penetrated the tablets. The tablets were then dried in a 50°C thermostatic chamber to obtain 5 mg tablets comprising cimetidine.

(Example 19)

100 mg of famotidine was dissolved in 600 mg of DMF to obtain a famotidine solution. 35 mg of the famotidine solution per tablet was dripped onto the tablets produced in Example 17 so that the solution penetrated the tablets. The tablets were then dried in a 50°C thermostatic chamber to obtain 5 mg famotidine impregnated tablets.

(Test Examples 17 to 19)

100 mg each of acetaminophen, cimetidine and famotidine were weighed into 100 ml flasks, dissolved in 50% methanol and then diluted to obtain standard solutions. The impregnated tablets prepared in Examples 17 to 19 were placed individually in measuring flasks, 50% methanol was added, and this was stirred to disintegrate the tablet. The resulting dispersion was centrifuged for 10 minutes at 10,000 rpm, and the supernatant was taken as a test solution. The absorbance of the standard solution and the test solution was measured with a UV-visible spectrophotometer, and the content was calculated based on the absorbance ratio. The measurement wavelengths of acetaminophen, cimetidine and famotidine were given as 246, 218 and 287 nm. The background measurement wavelength was 650 nm. The evaluation was performed on three of each kind of impregnated tablet, the average was calculated, and the contents of the acetaminophen impregnated tablets, cimetidine impregnated tablets and famotidine impregnated tablets were 99.3%, 97.6% and 100.0%, respectively.

(Example 20)

500 mg of acetaminophen was dissolved in 500 $\mu$l of dehydrated ethanol to prepare an acetaminophen solution. 20 $\mu$l of the acetaminophen solution was dripped onto the tablet for impregnation prepared in Example 17, which was then dried for 1 minute at 1000 W with a microwave (Sanyo Electric EMO-FZ40) to obtain an acetaminophen impregnated tablet.

(Test Example 20)

A Raman spectrometer (inVia, manufactured by Renishaw) was used to obtain an optical image and Raman microscope image of a cross-section of the acetaminophen impregnated tablet obtained in Example 20. Images were prepared at a laser wavelength of 785 nm with an exposure time of 1 second and an integration number of 1 using the intensity ratio between the signal and a baseline with a scattering intensity of 1595 to 1635 $cm^{-1}$ (Fig. 1). The part shown in white in the figure is an area where acetaminophen is present. The image shows a cross section perpendicular to the surface on which the acetaminophen solution was dripped on the tablet for impregnation, with the upper side of the image showing the dripping surface and the lower side of the image showing the surface opposite the dripping surface. This shows that the acetaminophen is present non-uniformly as a result of having moved from the dripping surface towards the opposite surface.

[Table 1]

| | | Reference Example 1 | Reference Example 2 | Reference Example 3 | Reference Example 4 | Example 1 | Example 2 | Example 3 | Example 4 | Example 5 | Example 6 | Example 7 | Example 8 | Example 9 | Example 10 | Example 11 |
|---|---|---|---|---|---|---|---|---|---|---|---|---|---|---|---|---|
| Monosaccharide or disaccharide | δ-form D-mannitol (Parteck Delta M) | 150 | 150 | 150 | 150 | 142,5 | 141 | 141 | 141 | 141 | 141 | 141 | 141 | 141 | 141 | 141 |
| | Sorbitol | | | | | | | | | | | | | | | |
| | Lactose hydrate | | | | | | | | | | | | | | | |
| | β-form D-mannitol (Parteck M) | | | | | | | | | | | | | | | |
| Water-insoluble polymer | Croscarmellose sodium | | | | | | | | | | 7,5 | | | | | |
| | Carmellose | | | | | | | | | | | 7,5 | | | | |
| | Crospovidone | | | | | | | | | | | | 7,5 | | | |
| | Microcrystalline cellulose | | | | | | | | | | | | | 7,5 | | |
| | Low-substituted hydroxypropyl cellulose (LH-31) | | | | | 7,5 | 7,5 | 7,5 | | | | | | | | 7,5 | 7,5 |
| | Low-substituted hydroxypropyl cellulose (LH-11) | | | | | | | | 7,5 | | | | | | | |
| | Low-substituted hydroxypropyl cellulose (LH-21) | | | | | | | | | 7,5 | | | | | | |
| Binder | (Partially saponified) polyvinyl alcohol | | | | | 1,5 | | 1,5 | 1,5 | 1,5 | 1,5 | 1,5 | 1,5 | | | |
| | Povidone (PVP) | | | | | | 1,5 | | | | | | | | | |
| | Hypromellose | | | | | | | | | | | | | | 1,5 | |
| | Pullulan | | | | | | | | | | | | | | | 1,5 |
| | Xanthan gum | | | | | | | | | | | | | | | |
| Solvent | Sterile purified water | 10% | 10% | 10% | 10% | 10% | 10% | 10% | 10% | 10% | 10% | 10% | 10% | 10% | 10% | 10% |
| | Ethanol anhydride | 10% | 10% | 10% | 10% | 10% | 10% | 10% | 10% | 10% | 10% | 10% | 10% | 10% | 10% | 10% |
| Total | | 150 | 150 | 150 | 150 | 150 | 150 | 150 | 150 | 150 | 150 | 150 | 150 | 150 | 150 | 150 |
| Evaluation | Permeation time (seconds) | 106 | 79 | 56 | 31 | 13 | 5 | 23 | 5 | 4 | 3 | 8 | 6 | 34 | 18 | 16 |
| | Retention ability | A | A | A | A | A | A | A | A | A | A | A | A | A | A | A |
| | Theoretical porosity (%) | 33 | 35 | 38 | 42 | 39 | 37 | 36 | 39 | 39 | 41 | 37 | 40 | 35 | 37 | 38 |
| | Thickness (mm) | 3,10 | 3,12 | 3,31 | 3,6 | 3,4 | 3,3 | 3,3 | 3,4 | 3,4 | 3,5 | 3,3 | 3,5 | 3,2 | 3,3 | 3,5 |
| | Diameter (mm) | 8,00 | 8,05 | 8,07 | 8,0 | 8,0 | 8,0 | 8,0 | 8,0 | 8,0 | 8,0 | 8,0 | 8,0 | 8,0 | 8,0 | 8,0 |
| | Hardness (N) | 54 | 52 | 71 | 62 | 26 | 25 | 40 | 16 | 11 | 15 | 26 | 16 | 35 | 25 | 15 |
| | Disintegration time (seconds) | 93 | 99 | 93 | 78 | 40 | 20 | 12 | 13 | 14 | 51 | 29 | 35 | 29 | 26 | 11 |

| | | Example 12 | Example 13 | Example 14 | Example 15 | Reference Example 5 | Example 16 | Reference Example 6 | Reference Example 7 | Reference Example 8 | Comparative Example 1 | Comparative Example 2 | Example 17 | Example 18 | Example 19 | Example 20 |
|---|---|---|---|---|---|---|---|---|---|---|---|---|---|---|---|---|
| Monosaccharide or disaccharide | δ-form D-mannitol (Parteck Delta M) | 141 | 133,5 | 139,5 | 150,4 | 148,5 | 150,4 | | | | 150,4 | | 150,7 | 150,7 | 150,7 | 150,7 |
| | Sorbitol | | | | | | | 141 | | | | | | | | |
| | Lactose hydrate | | | | | | | | 141 | | | | | | | |
| | β-form D-mannitol (Parteck M) | | | | | | | | | 141 | | | | | | |
| Water-insoluble polymer | Croscarmellose sodium | | | | | | | | | | | | | | | |
| | Carmellose | | | | | | | | | | | | | | | |
| | Crospovidone | | | | | | | | | | | | | | | |
| | Microcrystalline cellulose | | | | | | | | | | | 148,5 | | | | |
| | Low-substituted hydroxypropyl cellulose (LH-31) | 7,5 | 15 | 7,5 | 8 | | 8 | 7,5 | 7,5 | 7,5 | 8 | | 8,0 | 8,0 | 8,0 | 8,0 |
| | Low-substituted hydroxypropyl cellulose (LH-11) | | | | | | | | | | | | | | | |
| | Low-substituted hydroxypropyl cellulose (LH-21) | | | | | | | | | | | | | | | |
| Binder | (Partially saponified) polyvinyl alcohol | | 1,5 | 3 | 1,6 | 1,5 | 1,6 | 1,5 | 1,5 | 1,5 | 1,6 | 1,5 | 1,3 | 1,3 | 1,3 | 1,3 |
| | Povidone (PVP) | | | | | | | | | | | | | | | |
| | Hypromellose | | | | | | | | | | | | | | | |
| | Pullulan | | | | | | | | | | | | | | | |
| | Xanthan gum | 1,5 | | | | | | | | | | | | | | |
| Solvent | Sterile purified water | 10% | 10% | 10% | 10% | 10% | 20% | 10% | 10% | 10% | | 10% | 16% | 16% | 16% | 16% |
| | Ethanol anhydride | 10% | 10% | 10% | 10% | 10% | | 10% | 10% | 10% | 20% | 10% | 4% | 4% | 4% | 4% |
| Total | | 150 | 150 | 150 | 160 | 150 | 160 | 150 | 150 | 150 | 160 | 150 | 160 | 160 | 160 | 160 |
| Evaluation | Permeation time (seconds) | 15 | 4 | 9 | 14 | 76 | 67 | 102 | 8 | 12 | 7 | 66 | - | - | - | - |
| | Retention ability | A | A | A | A | B | B | B | B | B | A | C | A | A | A | A |
| | Theoretical porosity (%) | 38 | 39 | 36 | 34 | 31 | 30 | 30 | 34 | 35 | 35 | 34 | - | - | - | - |
| | Thickness (mm) | 3,3 | 3,4 | 3,3 | 3,4 | 3,0 | 3,2 | 2,8 | 3,1 | 3,2 | 3,4 | 3,2 | - | - | - | - |
| | Diameter (mm) | 8,0 | 8,0 | 8,0 | 8,0 | 8,0 | 8,0 | 8,2 | 8,0 | 8,0 | 8,0 | 7,9 | - | - | - | - |
| | Hardness (N) | 26 | 17 | 45 | 29 | 44 | 64 | 45 | 18 | 23 | 2 | 62 | - | - | - | - |
| | Disintegration time (seconds) | 24 | 29 | 49 | | 50 | | 201 | 41 | 50 | | - | - | - | - | - |

## Claims

1. A tablet for being impregnated with an active pharmaceutical ingredient, wherein the tablet comprises a monosaccharide and/or a disaccharide and a water-insoluble polymer, wherein the monosaccharide and/or disaccharide comprises $\delta$-form D-mannitol.

2. The tablet according to Claim 1, wherein the theoretical porosity of the tablet is from 20% to 50%, and wherein the theoretical porosity is determined in accordance with the method defined in the description.

3. The tablet according to Claim 1 or 2, wherein the water-insoluble polymer comprises at least one selected from the group consisting of croscarmellose sodium, carmellose, crospovidone, microcrystalline cellulose and low-substituted hydroxypropyl cellulose.

4. The tablet according to any one of Claims 1 to 3, wherein the content of the water-insoluble polymer is from 0.1 to 30% by mass based on the mass of the tablet.

5. The tablet according to any one of Claims 1 to 4, further comprising a binder, wherein the binder comprises preferably at least one selected from the group consisting of organic solvent insoluble binders, organic solvent hardly soluble binders, and polyvinyl pyrrolidone, and more preferably at least one selected from the group consisting of polyvinyl alcohol, pullulan, xanthan gum and hypromellose.

6. The tablet according to Claim 5, wherein the content of the binder is from 0.1 to 10% by mass based on the mass of the tablet.

7. A method for producing a tablet for being impregnated with an active pharmaceutical ingredient, comprising:

   a mixing step of mixing a monosaccharide and/or disaccharide and a water-insoluble polymer with a solvent to obtain a mixture; and
   a tableting step of tableting the mixture,
   wherein the monosaccharide and/or disaccharide comprises $\delta$-form D-mannitol.

8. The production method according to Claim 7, wherein the tableting step is performed on the mixture in a wet state.

9. The production method according to Claim 7 or 8, wherein the solvent comprises at least water.

10. The production method according to Claim 9, wherein the solvent further comprises an organic solvent.

11. The production method according to any one of Claims 7 to 10, wherein the mixture further comprises a binder.

12. A method for producing a medicine, comprising an impregnation step of impregnating the tablet according to any one of Claims 1 to 6 with an active pharmaceutical ingredient, wherein the impregnation step preferably comprises dripping the active pharmaceutical ingredient onto the tablet.

13. A medicine comprising the tablet according to any one of Claims 1 to 6 which is impregnated with an active pharmaceutical ingredient, wherein
the active pharmaceutical ingredient is preferably non-uniformly present in the tablet.

14. A kit comprising:

   the tablet according to any one of Claims 1 to 6; and
   an active pharmaceutical ingredient.

## Patentansprüche

1. Tablette zum Imprägnieren mit einem pharmazeutischen Wirkstoff, wobei die Tablette umfasst ein Monosaccharid und/oder ein Disaccharid und ein wasserunlösliches Polymer, wobei das Monosaccharid und/oder Disaccharid $\delta$-Form-D-Mannitol umfasst.

**2.** Tablette gemäß Anspruch 1, wobei die theoretische Porosität der Tablette zwischen 20 % und 50 % liegt und wobei die theoretische Porosität gemäß dem in der Beschreibung definierten Verfahren bestimmt wird.

**3.** Tablette gemäß Anspruch 1 oder 2, wobei das wasserunlösliche Polymer mindestens eines umfasst, ausgewählt aus der Gruppe, bestehend aus Croscarmellose-Natrium, Carmellose, Crospovidon, mikrokristalliner Cellulose und niedrig substituierter Hydroxypropylcellulose.

**4.** Tablette gemäß mindestens einem der Ansprüche 1 bis 3, wobei der Gehalt an wasserunlöslichem Polymer 0,1 bis 30 Massenprozent, bezogen auf die Masse der Tablette, beträgt.

**5.** Tablette gemäß mindestens einem der Ansprüche 1 bis 4, die ferner ein Bindemittel umfasst, wobei das Bindemittel vorzugsweise mindestens eines umfasst, ausgewählt aus der Gruppe, bestehend aus in organischen Lösungsmitteln unlöslichen Bindemitteln, in organischen Lösungsmitteln schwer löslichen Bindemitteln und Polyvinylpyrrolidon, und noch bevorzugter mindestens eines, ausgewählt aus der Gruppe, bestehend aus Polyvinylalkohol, Pullulan, Xanthangummi und Hypromellose.

**6.** Tablette gemäß Anspruch 5, wobei der Gehalt des Bindemittels 0,1 bis 10 Massenprozent, bezogen auf die Masse der Tablette, beträgt.

**7.** Verfahren zur Herstellung einer Tablette zum Imprägnieren mit einem pharmazeutischen Wirkstoff, umfassend:

einen Mischschritt, bei dem ein Monosaccharid und/oder Disaccharid und ein wasserunlösliches Polymer mit einem Lösungsmittel gemischt werden, um eine Mischung zu erhalten; und
einen Tablettierungsschritt, bei dem die Mischung zu Tabletten geformt wird,
wobei das Monosaccharid und/oder Disaccharid $\delta$-Form-D-Mannitol umfasst.

**8.** Verfahren zur Herstellung gemäß Anspruch 7, wobei der Tablettierungsschritt an der Mischung in einem feuchten Zustand durchgeführt wird.

**9.** Verfahren zur Herstellung gemäß Anspruch 7 oder 8, wobei das Lösungsmittel mindestens Wasser umfasst.

**10.** Verfahren zur Herstellung gemäß Anspruch 9, wobei das Lösungsmittel ferner ein organisches Lösungsmittel umfasst.

**11.** Verfahren zur Herstellung gemäß mindestens einem der Ansprüche 7 bis 10, wobei die Mischung ferner ein Bindemittel umfasst.

**12.** Verfahren zur Herstellung eines Arzneimittels, umfassend einen Imprägnierungsschritt, bei dem die Tablette gemäß mindestens einem der Ansprüche 1 bis 6 mit einem pharmazeutischen Wirkstoff imprägniert wird, wobei der Imprägnierungsschritt vorzugsweise das Auftropfen des pharmazeutischen Wirkstoffs auf die Tablette umfasst.

**13.** Arzneimittel, umfassend die Tablette gemäß mindestens einem der Ansprüche 1 bis 6, die mit einem pharmazeutischen Wirkstoff imprägniert ist, wobei
der pharmazeutische Wirkstoff vorzugsweise ungleichmäßig in der Tablette vorhanden ist.

**14.** Kit, umfassend:

die Tablette gemäß mindestens einem der Ansprüche 1 bis 6; und
einen pharmazeutischen Wirkstoff.

## Revendications

**1.** Comprimé destiné à être imprégné d'un principe actif, dans lequel le comprimé comprend un monosaccharide et/ou un disaccharide et un polymère insoluble dans l'eau, dans lequel le monosaccharide et/ou le disaccharide comprend le D-mannitol sous forme $\delta$.

**2.** Comprimé selon la revendication 1, dans lequel la porosité théorique du comprimé est comprise entre 20 % et 50 %, et dans lequel la porosité théorique est déterminée selon le procédé défini dans la description.

3. Comprimé selon la revendication 1 ou 2, dans lequel le polymère insoluble dans l'eau comprend au moins un élément choisi dans le groupe consistant en la croscarmellose sodique, la carmellose, la crospovidone, la cellulose microcristalline et l'hydroxypropylcellulose faiblement substituée.

4. Comprimé selon l'une quelconque des revendications 1 à 3, dans lequel la teneur en polymère insoluble dans l'eau est comprise entre 0,1 et 30 % en masse par rapport à la masse du comprimé.

5. Comprimé selon l'une quelconque des revendications 1 à 4, comprenant en outre un liant, dans lequel le liant comprend de préférence au moins un élément choisi dans le groupe consistant en les liants insolubles dans les solvants organiques, les liants difficilement solubles dans les solvants organiques, et la pyrrolidone de polyvinyle, et plus préférentiellement au moins un élément choisi dans le groupe consistant en l'alcool polyvinylique, le pullulan, la gomme de xanthane et l'hypromellose.

6. Comprimé selon la revendication 5, dans lequel la teneur en liant est comprise entre 0,1 et 10 % en masse par rapport à la masse du comprimé.

7. Procédé de fabrication d'un comprimé destiné à être imprégné d'un principe actif, comprenant :

   une étape de mélange d'un monosaccharide et/ou d'un disaccharide et d'un polymère insoluble dans l'eau avec un solvant pour obtenir un mélange ; et
   une étape de compactage du mélange,
   dans lequel le monosaccharide et/ou le disaccharide comprend le D-mannitol sous forme $\delta$.

8. Procédé de fabrication selon la revendication 7, dans lequel l'étape de compactage est effectuée sur le mélange à l'état humide.

9. Procédé de fabrication selon la revendication 7 ou 8, dans lequel le solvant comprend au moins de l'eau.

10. Procédé de fabrication selon la revendication 9, dans lequel le solvant comprend en outre un solvant organique.

11. Procédé de fabrication selon l'une quelconque des revendications 7 à 10, dans lequel le mélange comprend en outre un liant.

12. Procédé de fabrication d'un médicament, comprenant une étape d'imprégnation du comprimé selon l'une quelconque des revendications 1 à 6 avec un principe actif, dans lequel l'étape d'imprégnation comprend de préférence le fait de faire goutter le principe actif sur le comprimé.

13. Médicament comprenant le comprimé selon l'une quelconque des revendications 1 à 6, imprégné d'un principe actif, dans lequel
   le principe actif est de préférence présent de manière non uniforme dans le comprimé.

14. Kit comprenant :

   le comprimé selon l'une quelconque des revendications 1 à 6 ; et
   un principe actif.

[Fig. 1]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001213765 A
- WO 2007076874 A1
- US 2006134202 A1
- EP 2433620 A1

**Non-patent literature cited in the description**

- *AAPS PharmSciTech*, 2009, vol. 10 (4), 1388-1395